# EUROPEAN PATENT SPECIFICATION

(11) **EP 1 880 221 B1**
(45) Date of publication and mention of the grant of the patent: **22.09.2010**
(21) Application number: 06754992.3
(22) Date of filing: 03.05.2006
(51) Int. Cl.: G01N 33/68

(54) **METHODS FOR STRATIFYING HEART FAILURE**
VERFAHREN ZUR STRATIFIZIERUNG VON HERZINSUFFIZIENZ
PROCÉDÉ POUR LA STRATIFICATION DE L'INSUFFISANCE CARDIAQUE

(30) Priority: 09.05.2005 EP 05010043
(43) Date of publication of application: 23.01.2008
(73) Proprietor: F. Hoffmann-La Roche AG, 4070 Basel (CH); Roche Diagnostics GmbH, 68305 Mannheim (DE)
(72) Inventor: AMANN-ZALAN, Ildiko, 69469 Weinheim (DE); TRAUTH, Bernhard, 68163 Mannheim (DE); RIVERA OTERO, Jose Miguel Center of Investigations, E-46022 Valencia (ES)
(74) Representative: Dick, Alexander
(86) International application number: PCT/EP2006/062031
(87) International publication number: WO 2006/120152

(56) References cited:
- WO-A-00/45176
- WO-A-03/087819
- US-A1- 2004 077 027
- US-A1- 2004 096 988
- US-A1- 2004 132 013
- YEO KIANG-TECK J ET AL: "Multicenter evaluation of the Roche NT-proBNP assay and comparison to the Biosite Triage BNP assay." CLINICA CHIMICA ACTA, vol. 338, no. 1-2, December 2003 (2003-12), pages 107-115, XP002392499 ISSN: 0009-8981
- NG L L ET AL: "Community screening for left ventricular systolic dysfunction using plasma and urinary natriuretic peptides" JOURNAL OF THE AMERICAN COLLEGE OF CARDIOLOGY, vol. 45, no. 7, 5 April 2005 (2005-04-05), pages 1043-1050, XP004819685 ISSN: 0735-1097
- NG L L ET AL: "Diagnosis of heart failure using urinary natriuretic peptides." CLINICAL SCIENCE (LONDON, ENGLAND : 1979) FEB 2004, vol. 106, no. 2, February 2004 (2004-02), pages 129-133, XP002336339 ISSN: 0143-5221
- HOKAMAKI JUN ET AL: "Urinary biopyrrins levels are elevated in relation to severity of heart failure." JOURNAL OF THE AMERICAN COLLEGE OF CARDIOLOGY. 19 MAY 2004, vol. 43, no. 10, 19 May 2004 (2004-05-19), pages 1880-1885, XP002336340 ISSN: 0735-1097
- COLLINS SEAN P ET AL: "Diagnostic and prognostic usefulness of natriuretic peptides in emergency department patients with dyspnea." ANNALS OF EMERGENCY MEDICINE. APR 2003, vol. 41, no. 4, April 2003 (2003-04), pages 532-545, XP002336338 ISSN: 0196-0644
- HUNT P J ET AL: "IMMUNOREACTIVE AMINO-TERMINAL PRO-BRAIN NATRIURETIC PEPTIDE (NT-PROBNP): A NEW MARKER OF CARDIAC IMPAIRMENT" CLINICAL ENDOCRINOLOGY, BLACKWELL SCIENTIFIC PUBLICATIONS, OXFORD, GB, vol. 47, 1997, pages 287-296, XP000913471 ISSN: 0300-0664
- MAIR J ET AL: "THE IMPACT OF CARDIAC NATRIURETIC PEPTIDE DETERMINATION ON THE DIAGNOSIS AND MANAGEMENT OF HEART FAILURE" CLINICAL CHEMISTRY AND LABORATORY MEDICINE, WALTER DE GRUYTER UND CO, DE, vol. 39, no. 7, July 2001 (2001-07), pages 571-588, XP008024871 ISSN: 1434-6621
- ALAN H.B. WU ET AL.: CLINICAL CHEMISTRY, vol. 50, no. 5, 2004, pages 876-873,
- ALAN H.B. WU ET AL.: CLINICAL CHEMISTRY, vol. 50, no. 5, 2004, pages 876-873,

## Description

The present invention relates to methods for allocating heart failure in a subject to NYNA classes I, II, III or IV based on the amount of Brain natriuretic peptide (BNP) in a urine sample. Moreover, the present invention encompasses methods for predicting whether a subject is at risk of developing cardiovascular events accompanied heart failure based on the amount of BNP in a urine sample.

An aim of modem medicine is to provide personalized or individualized treatment regimens. Those are treatment regimens which take into account a patient's individual needs or risks. A particularly important risk is the presence of a cardiovascular complication, particularly an unrecognized cardiovascular complication. Cardiovascular complications, particularly heart diseases, are the leading cause of morbidity and mortality in the Western hemisphere. Cardiovascular complications can remain asymptomatic for long periods of time. Therefore, reliable diagnosis of the presence of a cardiovascular complication is more difficult and error-prone than generally believed (Svendstrup Nielsen, L., et al. (2003). N-terminal pro-brain natriuretic peptide for discriminating between cardiac and non-cardiac dyspnoea. The European Jounal of Heart Failure).

Natriuretic Peptides are known to be molecular markers for cardiovascular disease progression. WO 02/083913 discloses that the brain natriuretic peptide (BNP) and variants thereof in the blood may be used to predict near-term mortality and morbidity in patients suffering already from heart failure. In an attempt to diagnose cardiovascular diseases, in particular myocardial ischemia, WO 02/089657 also discloses various blood biomarkers which may be used as prognostic indicators for said diseases and disorders once diagnosed. Among others, BNP is mentioned as a suitable biomarker. Moreover, Hutfless et al. have shown that based on the BNP concentration in patient blood after cardiac surgery, it shall be possible to predict morbidity or mortality within a 30 day period after said surgery (Hutfless 2004, Utility of B-type natriuretic peptide in predicting postoperative complications and outcomes in patients undergoing heart surgery. J Am Coll Cardiol 43: 1873-9). However, the study results were according to the authors not highly significant. However, the use of natriuretic peptides as diagnostic markers was previously put into jeopardy because of the dependence of the level of theses peptides in various body fluids including urine from proper kidney function (McCullough 2003. B-Type Nariuretic Peptides: A Diagnostic Breakthrough for Clinicians. Reviews in Cardiovascular Medicine 4: 72-80.).

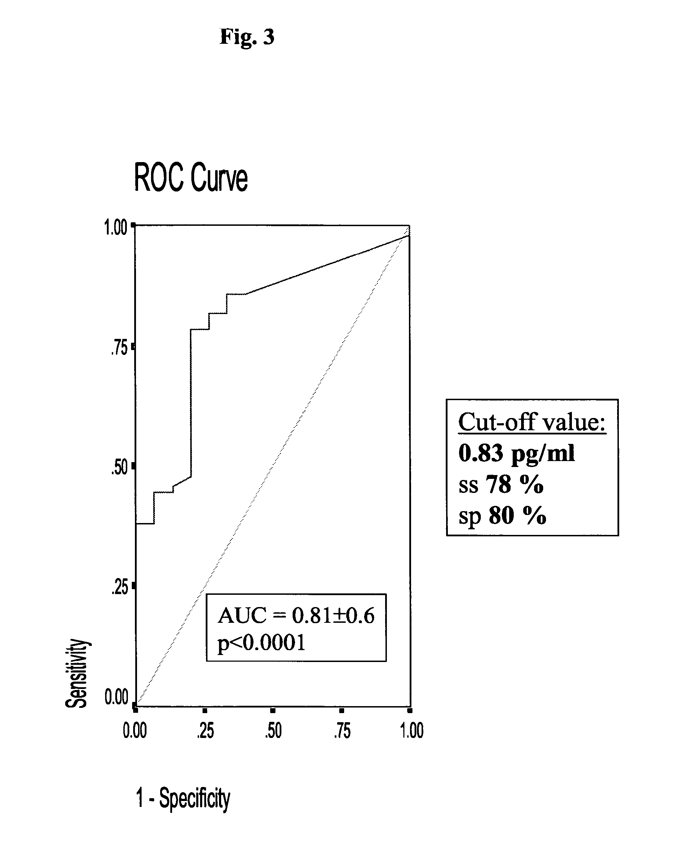

Wu and coworkers disclose a method for measuring the amount of BNP in a plasma sample from patients and assessing the NYHA classifications for these patients based on BNP amounts measured (see figure 3). The assay can be used for assessing the severity of heart failure because of the proportional increase in BNP concentrations of more than 2-fold when heart failure severity increased from NYHA class I to II, from class II to III, and from class III to IV (Alan H.B. Wu, et. al., Clinical Chemistry, 2004, Vol. 50(5) Pages 867-873).

WO 03/087819 A discloses a method for predicting a cardiac disease such as congestive heart failure by determining the levels of a BNP precursor or fragments thereof, such as the N-terminus of proBNP₁₋₂₁ in a sample such as urine.

A drawback of the prior art methods is that they require usually evaluation of the results by a specialized cardiologist or they may be only applicable after a cardiac event such as surgery happened already. Moreover, the reliability of the disclosed means and methods for diagnostic purposes, in particular for less severe forms of heart failure, is questionable due to the aforementioned uncertainties.

Therefore, there is a clear longstanding need for means and methods for diagnosing heart failure in patients which have not yet been identified to suffer from cardiovascular diseases or which have not been subjected to heart surgery. Moreover, the means and methods shall allow a reliable and rapid diagnosis. Specifically, they shall allow diagnosis of heart failure for a general practitioner and not only for a specialized clinician or cardiologist.

Thus, the technical problem underlying the present invention must be seen as the provision of methods for complying with the aforementioned needs. The methods shall, at the same time, avoid the drawbacks of the prior art referred to above. The technical problem is solved by the embodiments characterized in the claims and herein below.

Accordingly, the present application describes to a method for diagnosing heart failure in a subject comprising
a) determining the amount of Brain natriuretic peptide (BNP) in a urine sample of said subject; and
b) diagnosing whether the subject suffers from heart failure based on the amount of BNP determined in step a).

The method for diagnosing is, preferably, an in vitro method. Step b) referred to above, preferably, comprises correlation of the amount determined in step a) with a reference standard or several reference standards as described in detail below. It may further comprise processing the diagnostic raw data of the correlation into a diagnostic result which may need no further interpretation by a clinician. Preferably, step a) and/or b) may in total or in part be assisted by automation, e.g., by a suitable computer program for correlating and/or processing the data or robotic and sensory equipment for determining the amount in step a).

The term "diagnosing" as used herein refers to assessing the probability according to which a subject is suffering or will suffer from heart failure. As will be understood by those skilled in the art, such an assessment is usually not intended to be correct for 100% of the subjects to be diagnosed. The term, however, requires that a statistically significant portion of subjects can be diagnosed to suffer from heart failure or to have a risk of suffering from heart failure in the future. Whether a portion is statistically significant can be determined without further ado by the person skilled in the art using various well known statistic evaluation tools, e.g., determination of confidence intervals, p-value determination, Student's t-test, Mann-Whitney test, etc.. Details are found in Dowdy and Wearden, Statistics for Research, John Wiley & Sons, New York 1983. Preferred confidence intervals are at least 90%, at least 95%, at least 97%, at least 98% or at least 99 %. The p-values are, preferably, 0.1, 0.05, 0.01, 0.005, or 0.0001.

Diagnosing includes monitoring, confirmation, subclassification and prediction of the relevant disease, symptoms or risks therefor. Monitoring relates to keeping track of an already diagnosed disease, or complication, e.g. to analyze the progression of the disease or the influence of a particular treatment on the progression of disease or complication. Confirmation relates to the strengthening or substantiating a diagnosis already performed using other indicators or markers. Subclassification relates to further defining a diagnosis according to different subclasses of the diagnosed disease, e.g. defining according to mild and severe forms of the disease as discussed below in detail. Prediction relates to prognosing a disease or complication before other symptoms or markers have become evident or have become significantly altered. Predicting as used herein also, preferably, encompasses diagnosing prevalence for heart failure.

The term "urine" according to the present invention comprises total urine or fractions thereof. The fractions are peptide- or polypeptide-containing fractions of the urine. Samples of urine can be obtained by any method known in the art. The samples may be subjected to various known pre-treatments prior to applying the method of the present invention (i.e. processed urine samples).

The term "heart failure" as used herein refers to an impaired function of the heart. Heart failure is classified into a functional classification system according to the NYHA (New York Heart Association). Patients of Class I have no obvious symptoms of cardiovascular disease. Physical activity is not limited, and ordinary physical activity does not cause undue fatigue, palpitation, or dyspnea (shortness of breath). Echocardiography, however, might detect abnormalities in patients on exertion. Patients of class II have slight limitation of physical activity. They are comfortable at rest, but ordinary physical activity results in fatigue, palpitation, or dyspnea. Patients of class III show a marked limitation of physical activity. They are comfortable at rest, but less than ordinary activity causes fatigue, palpitation, or dyspnea. Patients of class IV are unable to carry out any physical activity without discomfort. They show symptoms of cardiac insufficiency at rest. If any physical activity is undertaken, discomfort is increased. Accordingly, patients can be divided into individuals showing no clinical symptoms and those with symptoms (e.g. dyspnea). Preferably, heart failure is chronic heart failure. Moreover, preferably, heart failure as referred to herein relates to mild or moderate heart failure. The symptoms accompanied with mild or moderate heart failure as meant herein are less severe, i.e. they are no apparent symptoms (e.g. for class I) or symptoms according to NYHA class II or III. Preferably, the term relates to congestive heart failure which may be caused by various underlying diseases or disorders. As mentioned before, mild or moderate heart failure relates to heart failure according to NYHA class I, II or III.

By investigation and monitoring of the symptoms, patients suffering from heart failure, specifically those suffering from mild or moderate heart failure, in light of weak or ambiguous symptoms, can hardly be classified. It has been found in accordance with the present invention that a urinary amount of BNP of at least 0.8 pg/ml is indicative for heart failure. More preferably, a subject which belongs into NYHA class I will have an amount of BNP equivalent to 0.8 pg/ml to 2.4 pg/ml, most preferably 1.7 pg/ml in the Advia Carteener assay (Bayer). Also more preferably, a subject which belongs into NYHA class II will have an amount of BNP equivalent to 2.5 pg/ml to 3.9, most preferably 2.8 pg/ml in the Advia Carteener Assay. Preferably, a subject which belongs into NYHA class III will have an amount of BNP equivalent to 4.0 to 12.0 pg/ml, most preferably 5.4 pg/ml in the Advia Carteneer Assay. An even higher amount (i.e. larger than 12.0 pg/ml) is indicative for NYHA class IV. The abbreviation "ml" as used herein above refers to the volume of the sample of the urine in millilitres. The limits of the aforementioned ranges and the values may vary statistically (see Example and figures, below). Moreover, the ranges may further vary due to the specific assay which is used for detection. However, it is to be understood that the methods of the present invention shall allow a determination of the urinary BNP values with essentially the same sensitivity and/or specificity as the assay described in the accompanying Example (Advia Carteneenr Assay).

Determining the amount of BNP can be achieved by all known means for determining the amount of a polypeptide or peptide in a sample. Said means comprise immunoassay devices and methods which may utilize labeled molecules in various sandwich, competition, or other assay formats. Said assays will develop a signal which is indicative for the presence or absence of BNP. Moreover, the signal strength can, preferably, be correlated directly or indirectly (e.g. reverse- proportional) to the amount of polypeptide present in a sample. Devices and methods, furthermore, encompass biosensors, optical devices coupled to immunoassays, biochips, analytical devices such as mass-spectrometers, NMR- analyzers, or chromatography devices. Further, methods include micro plate ELISA-based methods, fully-automated or robotic immunoassays (available for example on Elecsys™ analyzers), CBA (an enzymatic Cobalt Binding Assay, available for example on Roche-Hitachi™ analyzers), and latex agglutination assays (available for example on Roche-Hitachi™ analyzers). Determining the amount according to the present invention relates to measuring the amount or concentration, preferably semi-quantitatively or quantitatively. Measuring can be done directly or indirectly. Indirect measuring includes measuring of cellular responses, bound ligands, labels, or enzymatic reaction products. In the context of the present invention, amount also relates to concentration. It is evident, that from the total amount of a substance of interest in a sample of known size, the concentration of the substance can be calculated, and vice versa. Determination of the amount or concentration of the BNP in a sample can be achieved by various techniques.

In a preferred embodiment, the method for measuring the amount of BNP comprises the steps of (a) contacting a cell capable of a cellular response to the polypeptide with the polypeptide for an adequate period of time, (b) measuring the cellular response. For measuring cellular responses, the urine sample or processed urine sample is, preferably, added to a cell culture and an internal or external cellular response is measured. The cellular response may include the expression of a reporter gene or the secretion of a substance, e.g. a peptide, polypeptide, or a small molecule.

In another preferred embodiment, the method for measuring the amount of BNP comprises the steps of (a) contacting the polypeptide with a suitable substrate for an adequate period of time, (b) measuring the amount of product.

In another preferred embodiment, the method for measuring the amount of BNP comprises the steps of (a) contacting the polypeptide with a specifically binding ligand, (b) (optionally) removing non-bound ligand, (c) measuring the amount of bound ligand.

Other preferred methods for measurement may include measuring the amount of a ligand or agent binding specifically to the peptide or polypeptide of interest. Binding according to the present invention includes both covalent and non-covalent binding. A ligand according to the present invention can be any peptide, polypeptide, nucleic acid, or small molecule binding to BNP described herein. Preferably, the ligand or agent binds specifically to BNP. "Specific binding" according to the present invention means that the ligand or agent should not bind substantially to ("cross-react" with) another peptide, polypeptide or substance present in the sample to be analyzed. Preferably, the specifically bound protein should be bound with at least 3 times higher, more preferably at least 10 times higher and even more preferably at least 50 times higher affinity than any other relevant peptide or polypeptide. Non-specific binding may be tolerable, if can still be distinguished and measured unequivocally, e.g. according to its size on a Western Blot, or by its relatively higher abundance in the sample. Binding of the ligand can be measured by any method known in the art. Preferably, the method is semi-quantitative or quantitative. Suitable methods are described in the following. First, binding of a ligand may be measured directly, e.g. by NMR or surface plasmon resonance. Second, if the ligand also serves as a substrate of an enzymatic activity of the peptide or polypeptide of interest, an enzymatic reaction product may be measured (e.g. the amount of a protease can be measured by measuring the amount of cleaved substrate, e.g. on a Western Blot). For measurement of enzymatic reaction products, preferably the amount of substrate is saturating. The substrate may also be labeled with a detectable lable prior to the reaction. Preferably, the sample is contacted with the substrate for an adequate period of time. An adequate period of time refers to the time necessary for an detectable, preferably measurable amount of product to be produced. Instead of measuring the amount of product, the time necessary for appearance of a given (e.g. detectable) amount of product can be measured. Third, the ligand may be coupled covalently or non-covalently to a label allowing detection and measurement of the ligand. Labeling may be done by direct or indirect methods. Direct labeling involves coupling of the label directly (covalently or non-covalently) to the ligand. Indirect labeling involves binding (covalently or non-covalently) of a secondary ligand to the first ligand. The secondary ligand should specifically bind to the first ligand. Said secondary ligand may be coupled with a suitable label and/or be the target (receptor) of tertiary ligand binding to the secondary ligand. The use of secondary, tertiary or even higher order ligands is often used to increase the signal. Suitable secondary and higher order ligands may include antibodies, secondary antibodies, and the well-known streptavidin-biotin system (Vector Laboratories, Inc.). The ligand or substrate may also be "tagged" with one or more tags as known in the art. Such tags may then be targets for higher order ligands. Suitable tags include biotin, digoxygenin, His-Tag, Glutathion-S-Transferase, FLAG, GFP, myc-tag, influenza A virus haemagglutinin (HA), maltose binding protein, and the like. In the case of a peptide or polypeptide, the tag is preferably at the N-terminus and/or C-terminus. Suitable labels are any labels detectable by an appropriate detection method. Typical labels include gold particles, latex beads, acridan ester, luminol, ruthenium, enzymatically active labels, radioactive labels, magnetic labels ("e.g. magnetic beads", including paramagnetic and superparamagnetic labels), and fluorescent labels. Enzymatically active labels include e.g. horseradish peroxidase, alkaline phosphatase, beta-Galactosidase, Luciferase, and derivatives thereof. Suitable substrates for detection include di-amino-benzidine (DAB), 3,3'-5,5'-tetramethylbenzidine, NBT-BCIP (4-nitro blue tetrazolium chloride and 5-bromo-4-chloro-3-indolyl-phosphate, available as ready-made stock solution from Roche Diagnostics), CDP-Star™ (Amersham Biosciences), ECF™ (Amersham Biosciences). A suitable enzyme-substrate combination may result in a colored reaction product, fluorescence or chemoluminescence, which can be measured according to methods known in the art (e.g. using a light-sensitive film or a suitable camera system). As for measuring the enyzmatic reaction, the criteria given above apply analogously. Typical fluorescent labels include fluorescent proteins (such as GFP and its derivatives), Cy3, Cy5, Texas Red, Fluorescein, and the Alexa dyes (e.g. Alexa 568). Further fluorescent labels are available e.g. from Molcular Probes (Oregon). Also the use of quantum dots as fluorescent labels is contemplated. Typical radioactive labels include ³⁵S, ¹²⁵I, ³²P, ³³P and the like. A radioactive label can be detected by any method known and appropriate, e.g. a light-sensitive film or a phosphor imager. Suitable measurement methods according the present invention also include precipitation (particularly immunoprecipitation), electrochemiluminescence (electro-generated chemiluminescence), RIA (radioimmunoassay), ELISA (enzyme-linked immunosorbent assay), sandwich enzyme immune tests, electrochemiluminescence sandwich immunoassays (ECLIA), dissociation-enhanced lanthanide fluoro immuno assay (DELFIA), scintillation proximity assay (SPA), turbidimetry, nephelometry, latex-enhanced turbidimetry or nephelometry, or solid phase immune tests. Further methods known in the art (such as gel electrophoresis, 2D gel electrophoresis, SDS polyacrylamid gel electrophoresis (SDS-PAGE), Western Blotting, and mass spectrometry), can be used alone or in combination with labeling or other dectection methods as described above. Preferred ligands include antibodies, nucleic acids, peptides or polypeptides, and aptamers, e.g. nucleic acid or peptide aptamers. Methods to such ligands are well-known in the art. For example, identification and production of suitable antibodies or aptamers is also offered by commercial suppliers. The person skilled in the art is familiar with methods to develop derivatives of such ligands with higher affinity or specificity. For example, random mutations can be introduced into the nucleic acids, peptides or polypeptides. These derivatives can then be tested for binding according to screening procedures known in the art, e.g. phage display. Antibodies as referred to herein include both polyclonal and monoclonal antibodies, as well as fragments thereof, such as Fv, Fab and F(ab)₂ fragments that are capable of binding antigen or hapten. The present invention also includes humanized hybrid antibodies wherein amino acid sequences of a non-human donor antibody exhibiting a desired antigen-specificity are combined with sequences of a human acceptor antibody. The donor sequences will usually include at least the antigen-binding amino acid residues of the donor but may comprise other structurally and/or functionally relevant amino acid residues of the donor antibody as well. Such hybrids can be prepared by several methods well known in the art.

In another preferred embodiment, the ligand, preferably chosen from the group consisting of nucleic acids, peptides, polypeptides, antibodies and aptamers, is present on a solid support, preferably an array. Such solid supports or arrays comprising a ligand or binding agent for the BNP are well known in the art and include, inter alia, commercially available column materials, polystyrene beads, latex beads, magnetic beads, colloid metal particles, glass and/or silicon chips and surfaces, nitrocellulose strips, membranes, sheets, duracytes, wells and walls of reaction trays, plastic tubes etc. The ligand or agent may be bound to many different carriers. Examples of well-known carriers include glass, polystyrene, polyvinyl chloride, polypropylene, polyethylene, polycarbonate, dextran, nylon, amyloses, natural and modified celluloses, polyacrylamides, agaroses, and magnetite. The nature of the carrier can be either soluble or insoluble for the purposes of the invention. Suitable methods for fixing/immobilizing said ligand or agent are well known and include, but are not limited to ionic, hydrophobic, covalent interactions and the like. It is also contemplated to use "suspension arrays" as arrays according to the present invention (Nolan JP, Sklar LA. (2002). Suspension array technology: evolution of the flat-array paradigm. Trends Biotechnol. 20(1):9-12). In such suspension arrays, the carrier, e.g. a microbead or microsphere, is present in suspension. The array consists of different microbeads or microspheres, possibly labeled, carrying different ligands. Methods of producing such arrays, for example based on solid-phase chemistry and photo-labile protective groups, are generally known (US 5,744,305). Such arrays can also be brought into contact with substances or substance libraries and tested for interaction, for example for binding or change of confirmation. Therefore, arrays comprising a peptide or polypeptide as defined above may be used for identifying ligands binding specifically to said peptides or polypeptides.

The term "BNP" encompasses mature BNP. The term "variants" encompasses also allelic variants encoded by homologous genes either in human or other animal species. Such homologous genes are, preferably, paralogous or orthologous genes encoding the aforementioned polypeptides. Preferred BNPs and variants thereof to be applied in accordance with the present invention are described below in detail. The terms "peptide" and "polypeptide" may be both used interchangeable in this specification.

Diagnosing whether the subject suffers from mild or moderate heart failure based on the amount of BNP comprises correlating the determined amount of BNP with a reference amount. The reference amount will be either determined simultaneously or will be determined separately. In the later case, the reference will be stored or recorded in a suitable manner and provided for the correlation. The reference amount will be indicative for heart failure. In one embodiment, the reference amount will be an amount determined from a subject or group of subjects (i. e. an average or mean value of said group) known not to suffer from heart failure, i.e. healthy subject(s). Said healthy subject(s) are characterized in that they appear normal in echo-Doppler studies, show normal electrocardiograms (ECG), and show normal ranges for standard biochemical and haematological parameters. Moreover, the healthy subjects shall not show any of the symptoms according to the NYHA classification. Diagnosis will be made by correlating the determined amount with the said reference amount whereby an increase in amount in comparison to the reference amount is indicative for heart failure.

The determined amount is compared to reference amounts from reference subjects or groups thereof known to suffer from heart failure. Diagnosis will be made by correlating the determined amount with the said reference amount whereby a similar or increased amount in comparison to the reference amount is indicative for heart failure.

A panel of reference values will be provided, each correlating with a different strength of symptoms associated with heart failure, i.e. an individual reference value will be provided for each of the heart failures according to NYHA class I, II, III and IV. Preferred reference values have been discussed already above. The symptoms according to the standards of the NYHA have been discussed already elsewhere in the specification in detail. According to this embodiment, it is not only possible to diagnose whether a subject suffers from heart failure. Rather, it is also possible to diagnose the strength of the symptoms which a given subject will develop. Thus, subjects suffering from heart failure can be also reliably identified and allocated into a respective NYHA class.

Thanks to the present invention it is possible to allocate heart failure in NYHA classes, or a risk of developing cardiovascular events accompanied with heart failure by using a test device which is easy to handle and independent on the evaluation by a highly specialized clinician. Thus, depending on the method, a clinician may no longer be required for making a diagnosis. For a general practitioner, expensive diagnostic tools are no longer required for making cardiac diagnosis. Advantageously, using urine as sample avoids any invasive step performed on the subject. Surprisingly, it has been found in the studies underlying the invention that the amount of BNP in urine is at least as valuable and reliable for diagnosis as the amount in the blood. Usually, one would expect merely minor levels of BNP to be present in urine since the major clearance for said polypeptide takes place in the kidney by glomerular filtration. Accordingly, one would not expect that (i) sufficient amounts of BNP are at all present in urine and (ii) that a reliable diagnosis or prediction can be based thereon because the amount of BNP in urine is also dependent on proper glomerular filtration. Moreover, it has been found in the studies underlying this invention that the amount of BNP present in a urine sample is indicative not only for the development of heart failure but also allows reliable classification said heart failure according to the NYHA classification systems. This allows, furthermore, efficient monitoring of disease progression. The methods described herein may be applied in studies relating to drug efficacy. Specifically, it is possible to reliably determine whether a drug or drug candidate ameliorates heart failure, diseases accompanied therewith or the symptoms referred to herein. Thus, the method of the present invention will also facilitate clinical trials or studies.

The definitions and explanations of the terms made above apply for the embodiments described in the following mutatis mutandis. The same applies for all definitions and explanations given below.

Moreover, the present invention describes a device for diagnosing heart failure in a subject comprising
a) means for determining the amount of BNP in a urine sample of said subject; and
b) means for diagnosing whether the subject suffers from heart failure based on the amount of BNP.

The term "device" as used herein relates to a system of means comprising at least the aforementioned means operatively linked to each other as to allow diagnosis of heart failure. Preferred means for determining the amount of BNP and for diagnosing heart failure are disclosed above in connection with the method of the invention. How to link the means in an operating manner will depend on the type of means included into the device. For example, where means for automatically determining the amount of BNP are applied, the data obtained by said automatically operating means can be processed by, e.g., a computer program in order to diagnose heart failure. Preferably, the means are comprised by a single device in such a case. Said device may accordingly include an analyzing unit for the measurement of the amount of BNP in an applied sample and a computer unit for processing the resulting data for the diagnosis. Alternatively, where means such as test stripes are used for determining the amount of BNP, the means for diagnosing may comprise control stripes or tables allocating the determined amount to an amount known to be accompanied with heart failure or an amount known to be indicative for a healthy subject as discussed above. The test stripes are, preferably, coupled to a ligand or agent which specifically binds to BNP. The strip or device, preferably, comprises means for detection of the peptide derived from preproBNP binding to the said ligand or agent. Preferred means for detection are disclosed in connection with embodiments relating to the method of the invention above. In such a case, the means are operatively linked in that the user of the system brings together the result of the determination of the amount and the diagnostic value thereof due to the instructions and interpretations given in a manual. The means may appear as separate devices in such an embodiment and are, preferably, packaged together as a kit. The person skilled in the art will realize how to link the means without further ado. Preferred devices are those which can be applied without the particular knowledge of a specialized clinician, e.g., test stripes or electronic devices which merely require loading with a sample. The results may be given as output of diagnostic raw data which need interpretation by the clinician. Preferably, the output of the device are, however, processed diagnostic raw data the interpretation of which does not require a clinician, i.e. it should be inevitably clear from the output whether the subject suffers from heart failure. Further preferred devices comprise the analyzing units/devices (e.g., biosensors, arrays, solid supports coupled to ligands or agents specifically recognizing BNP, Plasmon surface resonace devices, NMR spectrometers, mass- spectrometers etc.) or evaluation units/devices referred to above in accordance with the method of the invention.

In light of the foregoing, in a preferred embodiment of the method of the present invention, said diagnosing based on the amount of BNP comprises comparing the amount of BNP in a urine sample of the subject with the amount of BNP in a urine sample of a subject known not to suffer from heart failure or known to suffer from heart failure.

The present invention also relates to a method for predicting whether a subject is at risk of developing cardiovascular events after heart failure comprising
a) determining the amount of BNP in a urine sample of said subject; and
b) predicting whether the subject is at risk of developing cardiovascular events accompanied with heart failure based on the amount of BNP determined in step a).

The term "cardiovascular events" relates to cardiovascular death plus hospital admission for any cardiovascular event - heart failure, acute coronary syndrome, cerebrovascular accident/transient ischaemic attack, peripheral vascular event, arrhythmia or syncope. Moreover, any new out-patient episode of decompensated heart failure requiring an increase in medication can me summarized under cardiovascular event as meant herein as well. The symptoms and diagnostic features of cardiovascular events are well known to the clinician (see e.g., Troughton 2000. Treatment of heart failure guided by plasma aminoterminal brain natriuretic peptide (N-BNP) concentrations. The Lancet, Vol. 355.). Details are also described in standard text books such as Pschyrembel or Stedman. The symptoms and features can be determined and can be evaluated by the clinician by taking into account further parameters such as age, weight, size, and further diseases or disorders, therapies and/or clinical history of the subject. Preferably, the said cardiovascular events require hospitalization of the subject and/or are accompanied by mortality or increased morbidity of the subject.

It has been found in accordance with the present invention that, a urinary amount of BNP of at least 2.67 pg/ml is indicative for an increased risk of developing cardiovascular events and/or mortality (cardiac death) in the future. It is to be understood that the aforementioned values may vary within statistic boundaries.

Accordingly, the method of the present invention may be used for risk stratification for a patient. Thus, therapies or other necessary medical treatments or means may be applied to a subject. Moreover, the risk for developing heart failure may be taken into account for future therapies or medical means.

It follows from the foregoing, that in a preferred embodiment of the method of the invention, said predicting based on the amount of BNP comprises comparing the amount of BNP in a urine sample of the subject with the amount of BNP in a urine sample of a subject known not to develop heart failure or a subject known to develop heart failure.

In a further preferred embodiment of the methods of the invention, said risk of developing cardiovascular events accompanied with heart failure relates to a prognostic period of at least 12 months.

It has been found in the studies underlying the present invention that the prediction of cardiovascular events based on the amount of BNP is most reliable and significant within a prognostic period of 12 month. Further prognostic periods which are also envisaged by the present invention are periods of at least one year.

In another preferred embodiment of the methods of the invention, said heart failure can be allocated to New York Heart Association (NYHA) classes I, II, III or IV.

As discussed above, allocation of the mild or moderate heart failure into the NYHA classes can be performed based on the amount of BNP, i.e. the strength of the symptoms which a subject will develop can be determined based on the amount of BNP. In the studies underlying the present invention it has been surprisingly found that even patients which belong, in particular, into the NYHA classes I and II can be reliably allocated to their respective classes. Thereby, symptoms which may be developed by a subject can be predicted more reliably with respect to mild or moderate heart failure. Moreover, based on the knowledge of symptoms which a subject may develop, the subject's life can be made more convenient.

In a preferred embodiment of the methods of the invention, said subject is a human.

The human BNP as referred to in accordance with the present invention is a polypeptide comprising 32 amino acids in length corresponding to the mature molecule (amino acids 77 to 108 of preproBNP). The structure of the polypeptides has been described already in detail in the prior art, e.g., WO 02/089657, WO 02/083913, Bonow 1996, New Insights into the cardiac natriuretic peptides. Circulation 93: 1946-1950. The aforementioned prior art documents are herewith incorporated by reference with respect to the specific sequences of BNP and variants thereof disclosed therein. The BNPs referred to in accordance with the present invention further encompasses allelic. Specifically, envisaged are, thus, variant polypeptides which are on the amino acid level at least 60 % identical, more preferably at least 70 %, at least 80 %, at least 90 %, at least 95 %, at least 98% or at least 99 % identical to human BNP. Also encompassed are variant polypeptides having amino acid deletions or substitutions compared to human BNP, wherein said polypeptides have biological or immunological properties of human BNP. These variant polypeptides have the same predictive and diagnostic value with respect to heart failure as the specific BNPs referred to herein. BNP can be assayed, preferably, as described in WO 02/089657 or in the accompanying Example below.

Most preferably, the BNP is human BNP as specified above.

The figures show:

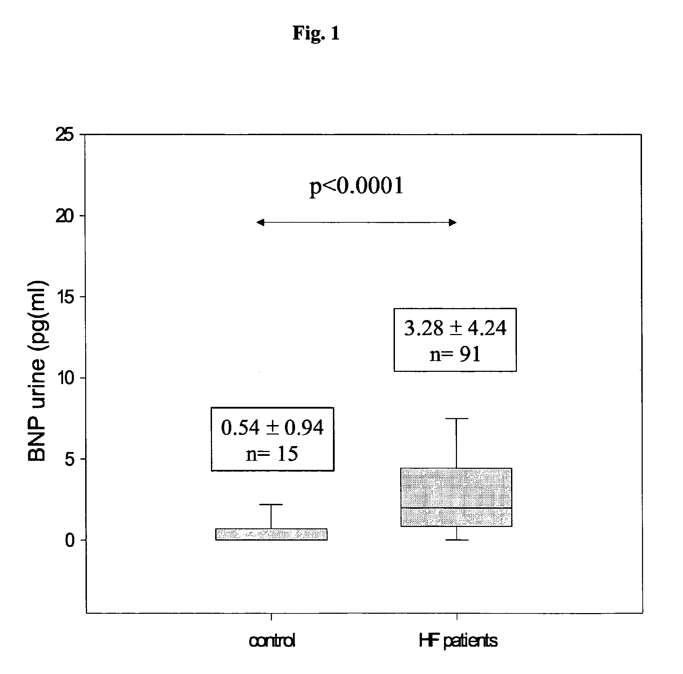

- Figure 1: Figure 1 shows a comparison of the amount of BNP to be found in urine of patients suffering from heart failure (HF patiens) and healthy controls. The p-value of p < 0.001 indicates that the difference found in the amounts is highly significant.

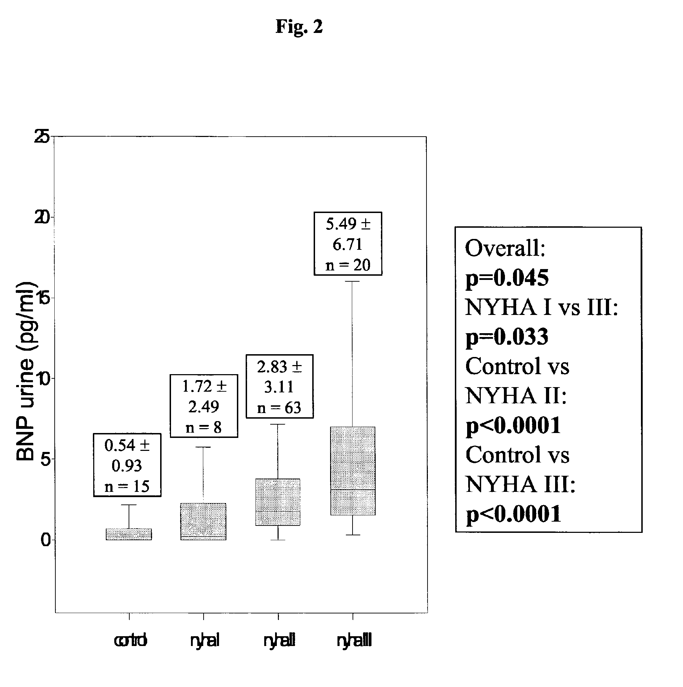

- Figure 2: Figure 2 shows different amounts of BNP in urine to be found in either healthy control individuals or individuals suffering from either heart failure according to the New York Heart Association Classes I, II or III (NYHA I, II or III). As is evident from the figure, the amount of BNP found in the urine of the individuals may be used to distinguish between the severeness of heart failure. The differences between the groups were statistically significant.
- Figure 3: Receiver operating curve (ROC) of the urinary BNP amount for the diagnosis of heart failure.

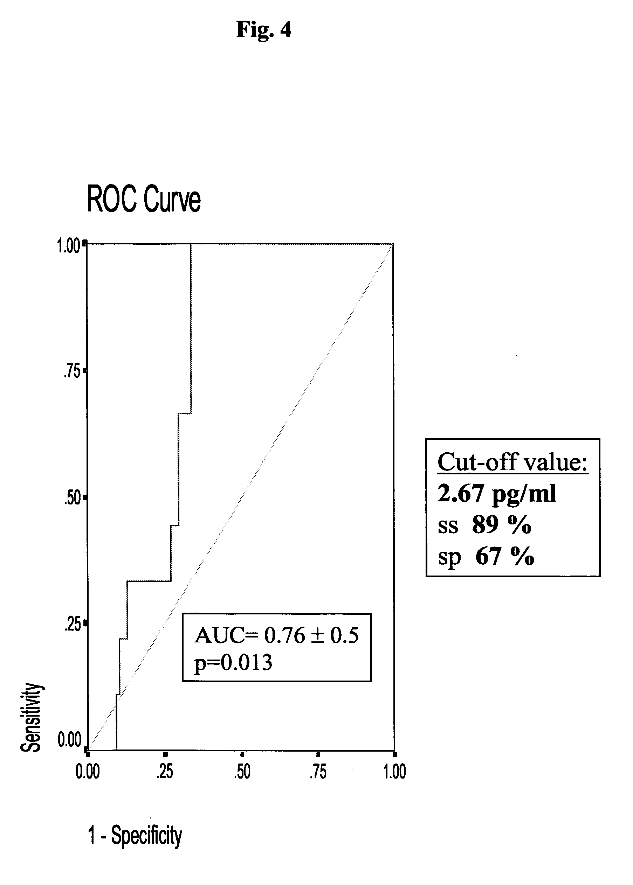

- Figure 4: Receiver operating curve (ROC curve) of urinary BNP amounts for the prognosis of cardiac death within a 12-months-time window after sample measurement

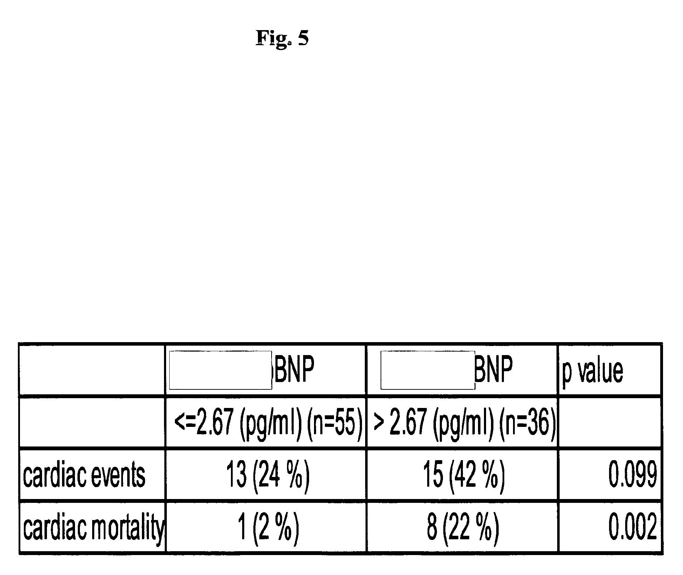

- Figure 5: Figure 5 shows a table with the most preferred cut-off values of the mortality ROC curve found in the study underlying the present invention.

The following Example illustrates the invention.

### Example: Diagnostic and prognostic value of urinary BNP

The aim of the current investigation was to analyze the presence of BNP in human urine, to compare the levels found between controls and a diseased group and to investigate the diagnostic and prognostic value of BNP in urine. Urine BNP levels were measured in 15 healthy volunteers and 91 Heart failure (HF) patients. Among the HF group 8 were in NYHA states I, 63 in NYHA states II and 20 in NYHA states III.

Urine BNP levels were statistically significant higher in HF patients than in controls (3.28 ± 4.24 vs. 0.54 ± 0.94 pg/ml; p < 0.0001). In deteriorated functional classes mean BNP urine levels were significantly elevated (Fig. 3) according to an increase of severity. The ROC curve of BNP for detection of HF yielded an area under the curve (AUC) of 0.81; p < 0.0001 compared with the diagonal. From this ROC curve the optional cut-point in this specific assay would be 0.83 pg/ml with a specificity of 80 % and a sensitivity of 78 %. Furthermore, to investigate whether urinary BNP was prognostic for cardiac death (12 months) a ROC curve was performed yielding an AUC = 0.76 p = 0.013 compared to the diagonal. The optimal cut-point to prognose cardiac mortality was calculated to be 2.67 pg/ml in this particular assay.

Thus, the current investigations show that BNP can be determined in human urine samples and that BNP levels in urine can be used for the diagnosis and the prognosis of heart failure patients as well as for the characterization of the functional states of these patients.

## Claims

1. Method for allocating heart failure in a subject to New York Heart Association (NYHA) classes I, II, III or IV comprising
a) determining the amount of the 32 amino acid mature Brain natriuretic peptide (BNP) in a urine sample of said subject; and
b) allocating whether the subject suffers from heart failure according to NYHA class I, II, III or IV based on the amount of the 32 amino acid mature BNP determined in step a), wherein
i. an amount of 0.8 to 2.4 pg/ml allocates a subject to NYHA class I,
ii. an amount of 2.5 to 3.9 pg/ml allocates a subject to NYHA class II,
iii. an amount of 4.0 to 12.0 pg/ml allocates a subject to NYHA class III; and
iv. an amount above 12.0 pg/ml allocates a subject to NYHA class IV.

2. Method for predicting whether a subject is at risk of developing cardiovascular events accompanied with heart failure comprising
a) determining the amount of the 32 amino acid mature BNP in a urine sample of said subject; and
b) predicting whether the subject is at risk of developing cardiovascular events accompanied with heart failure based on the amount of the 32 amino acid mature BNP determined in step a); wherein an amount of BNP of at least 2.67 pg/ml is indicative for an increased risk of developing cardiovascular events.

3. The method of claim 2, wherein said risk of developing cardiovascular events accompanied with heart failure relates to a prognostic period of 12 months.

4. The method of any one of claims 2 to 3, wherein said heart failure is heart failure according to NYHA class I, II or III.

5. The method of any one of claims 1 to 4, wherein said heart failure is chronic heart failure.

6. The method of any one of claims 1 to 5, wherein said subject is a human.

7. Use of the 32 amino acid mature BNP in a urine sample of a subject for allocating heart failure to NYHA classes, I, II, III or IV.

8. Use of the 32 amino acid mature BNP in a urine sample of a subject for predicting whether said subject is at risk of developing heart failure.

## Patentansprüche

1. Verfahren zur Einordnung von Herzinsuffizienz bei einem Probanden nach den New York Heart Asscociation (NYHA)-Klassen I, II, III oder IV umfassend
a) Bestimmen der Menge des 32 Aminosäuren langen reifen natriuretischen Peptids aus dem Gehirn ("Brain Natriuretic Peptide", BNP) in einer Urinprobe des Probanden und
b) Einordnen, ob der Proband an Herzinsuffizienz nach NYHA-Klasse I, II, III oder IV leidet, basierend auf der Menge des 32 Aminosäuren langen reifen BNPs wie in Schritt a) bestimmt, wobei i) eine Menge von 0,8-2,4 pg/ml den Probanden in die NYHA-Klasse I einordnet, ii) eine Menge von 2,5-3,9 pg/ml den Probanden in die NYHA-Klasse II einordnet, iii) eine Menge von 4,0-12,0 pg/ml den Probanden in die NYHA-Klasse III einordnet und iv) eine Menge oberhalb von 12,0 pg/ml den Probanden in die NYHA-Klasse IV einordnet.

2. Verfahren zur Vorhersage, ob ein Proband ein Risiko für die Entwicklung eines kardiovaskulären Ereignisses, das mit Herzinsuffizienz einhergeht aufweist, umfassend
a) Bestimmen der Menge des 32 Aminosäure langen reifen BNP in einer Urinprobe des besagten Probanden und
b) Vorhersage, ob der Proband ein Risiko für die Entwicklung eines kardiovaskulären Ereignisses das mit Herzinsuffizienz einhergeht aufweist, basierend auf der Menge des 32 Aminosäure langen reifen BNPs bestimmt in Schritt a) wobei eine Menge von BNP von zumindest 2,76 pg/ml ein erhöhtes Risiko für die Entwicklung eines kardiovaskulären Ereignisses anzeigt.

3. Verfahren nach Anspruch 2, wobei das Risiko für die Entwicklung eines kardiovaskulären Ereignisses das mit Herzinsuffizienz einhergeht eine Vorhersageperiode von 12 Monaten betrifft.

4. Verfahren nach einem der Ansprüche 2 oder 3, wobei die Herzinsuffizienz eine Herzinsuffizienz gemäß NYHA-Klasse I, II oder III ist.

5. Verfahren nach einem der Ansprüche 1-4, wobei die Herzinsuffizienz chronische Herzinsuffizienz ist.

6. Verfahren nach einem der Ansprüche 1-5, wobei der Proband ein Mensch ist.

7. Verwendung des 32 Aminosäure langen reifen BNPs in einer Urinprobe eines Patienten zur Einordnung von Herzinsuffizienz gemäß NYHA-Klassen I, II, III oder IV.

8. Verwendung des 32 Aminosäure langen reifen BNPs in einer Urinprobe eines Probanden zur Vorhersage ob der Proband ein Risiko für die Entwicklung von Herzinsuffizienz aufweist.

## Revendications

1. Procédé d'attribution de l'insuffisance cardiaque d'un sujet aux classes I, II, III ou IV de la New York Heart Association (NYHA) comprenant
a) la détermination de la quantité de peptide B-natriurétique mature (BNP) de 32 acides aminés dans un échantillon d'urine dudit sujet ; et
b) l'attribution du fait que le sujet souffre d'insuffisance cardiaque selon la classe I, II, III ou IV de la NYHA sur la base de la quantité de BNP mature de 32 acides aminés déterminée dans l'étape a), dans lequel
i. une quantité de 0,8 à 2,4 pg/ml attribue un sujet à la classe I de la NYHA,
ii. une quantité de 2,5 à 3,9 pg/ml attribue un sujet à la classe II de la NYHA,
iii. une quantité de 4,0 à 12,0 pg/ml attribue un sujet à la classe III de la NYHA ; et
iv. une quantité située au-dessus de 12,0 pg/ml attribue un sujet à la classe IV de la NYHA.

2. Procédé de prédiction du fait qu'un sujet court le risque ou non de développer des événements cardiovasculaires accompagnés d'insuffisance cardiaque comprenant
a) la détermination de la quantité de BNP mature de 32 acides aminés dans un échantillon d'urine dudit sujet ; et
b) la prédiction du fait que le sujet court un risque ou non de développer des événements cardiovasculaires accompagnés d'insuffisance cardiaque sur la base de la quantité de BNP mature de 32 acides aminés déterminée dans l'étape a) ; dans lequel une quantité de BNP d'au moins 2,67 pg/ml est indicatrice d'un risque accru de développer des événements cardiovasculaires.

3. Procédé selon la revendication 2, dans lequel ledit risque de développer des événements cardiovasculaires accompagnés d'insuffisance cardiaque concerne une période prognostique de 12 mois.

4. Procédé selon l'une quelconque des revendications 2 à 3, dans lequel ladite insuffisance cardiaque est l'insuffisance cardiaque selon la classe I, II ou III de la NYHA.

5. Procédé selon l'une quelconque des revendications 1 à 4, dans lequel ladite insuffisance cardiaque est l'insuffisance cardiaque chronique.

6. Procédé selon l'une quelconque des revendications 1 à 5, dans lequel ledit sujet est un être humain.

7. Utilisation du BNP mature de 32 acides aminés dans un échantillon d'urine d'un sujet pour attribuer l'insuffisance cardiaque aux classes I, II, III ou IV de la NYHA.

8. Utilisation du BNP mature de 32 acides aminés dans un échantillon d'urine d'un sujet pour prédire le fait que ledit sujet court un risque ou non de développer une insuffisance cardiaque.
